# DEMANDE DE BREVET EUROPEEN

(11) **EP 2 158 877 A1**
(43) Date de publication de la demande: **03.03.2010**
(21) Numéro de dépôt: 09168993.5
(22) Date de dépôt: 31.08.2009
(51) Int. Cl.: A61F 2/34, A61F 2/40

(54) **Cotyle prothétique articulaire**

(30) Priorité: 01.09.2008 FR 0855838
(71) Demandeur: Pichon, Denis, 35310 Mordelles (FR)
(72) Inventeur: Pichon, Denis, 35310, MORDELLES (FR); Laine, Marc, 03400, YZEURE (FR); Viale, Patrick, 18000, BOURGES (FR)
(74) Mandataire: Bomer, Françoise Marie

(57) **Abrégé**

Ce cotyle (1) comprend une cupule rigide (2) adaptée pour fixée dans l'os, dont la cavité (5) présente une zone de fond bordée par une embouchure (6) conique, s'évasant vers l'extérieur, une coque (3) emboîtable dans cette cupule (2), dont 1a cavité (33) est destinée à recevoir la tête d'une prothèse articulaire, et qui est munie sur sa face externe, d'au moins une saillie (7) déformable. Il est remarquable en ce que, la coque (3) étant emboîtée dans la cupule (2), sa portion de paroi externe (31), tournée vers l'intérieur de la cupule (2), est entourée d'un espace annulaire libre situé en regard de la paroi d'embouchure conique (6), tandis que 1a saillie (7) est positionnée en vis-à-vis de la base de cette embouchure, une bague de blocage (4) pouvant être été emmanchée dans cet espace libre, afin de déformer 1a saillie (7) et d'assurer la solidarisation par friction de la coque (3) avec la paroi de la cavité (5) de'la cupule.

Chirurgie orthopédique, notamment pour l'arthroplastie de la hanche ou de l'épaule.

## Description

La présente invention a pour objet un cotyle prothétique articulaire.

Elle trouve notamment application dans l'arthroplastie totale de la hanche ou de l'épaule.

Une prothèse totale de l'articulation de la hanche, qui est mise en place dans une telle opération, comprend essentiellement deux parties, l'une « mâle » destinée à se substituer à la tête du fémur, l'autre « femelle » destinée à être logée dans la cavité cotyloïdienne du bassin, dans laquelle était insérée la tête du fémur.

La partie mâle comporte une tige apte à être implantée dans le canal médullaire de l'extrémité proximale du fémur, ainsi qu'une tête quasi sphérique, tandis que la partie femelle comporte un cotyle apte à être inséré et fixé, à l'aide de plusieurs vis et/ou au moyen d'un ciment approprié, dans la cavité naturelle de l'os du bassin.

Ce cotyle prothétique a la configuration générale d'une cupule dont la paroi externe convexe est adaptée à celle de la cavité naturelle du bassin, tandis que sa paroi interne concave présente une forme sensiblement hémisphérique, complémentaire de la tête de la partie mâle.

Après insertion et ancrage de cette dernière dans le cotyle prothétique, on obtient une articulation à rotule, stable et durable, dont les caractéristiques fonctionnelles sont proches de celles de l'articulation naturelle de la hanche.

Pour des raisons de coût et de gestion des stocks, il est intéressant de prévoir un cotyle à caractère modulaire, comprenant une cupule externe dure -dite acétabulaire- qui est en matériau biocompatible et est conformée de façon à pouvoir être insérée et convenablement fixée dans la cavité cotyloïdienne de l'os du bassin, ainsi qu'un insert interne interchangeable en forme de coque, pourvu d'une cavité sensiblement hémisphérique destinée à recevoir la tête de la prothèse fémorale, et assurer un contact glissant, à faible coefficient de frottement, avec cette dernière.

Il convient de noter que les couples de matériaux utilisés au niveau de cette interface de frottement tête de fémur/coque sont généralement les suivants : métal/métal, métal/polyéthylène, céramique/céramique, céramique/polyéthylène, métal/composite composite/céramique, ou céramique/composite.

La cupule externe est généralement en métal, par exemple en alliage de chrome/cobalt ou de titane.

En fonction des circonstances, qui peuvent notamment dépendre de la morphologie, du poids, de l'âge et de l'activité du patient concerné, le chirurgien est amené à choisir un diamètre donné pour la tête fémorale ainsi qu'un couple de matériaux tête de fémur/insert donné, parmi ceux énoncés ci-dessus.

Dans le domaine des prothèses orthopédiques, il est relativement courant d'assurer la liaison entre deux pièces par un emmanchement par un cône de faible pente, dit « cône morse ».

Les deux pièces à assembler, l'une mâle, l'autre femelle, possèdent des parois emboîtables à formes tronconiques complémentaires d'angle au sommet relativement faible, à titre indicatif compris entre 2 et 10° environ (soit un demi-angle au sommet compris entre 1 et 5° environ).

Ce mode de liaison est à la fois simple à réaliser et très efficace, assurant un très bon centrage et une solidarisation sûre des deux pièces l'une à l'autre, ceci aussi bien en rotation qu'en translation.

Une telle liaison peut être prévue aussi bien du côté fémur, pour assurer la fixation de la tête sur le col de la tige médullaire (voir par exemple le document FR 2 678 162), que du côté bassin, pour assurer la fixation de la coque dans la cupule de cotyle (voir par exemple le document US 5 919 236).

Une liaison du type cône morse est réellement efficace lorsque chacune des deux pièces à assembler, en l'occurrence la cupule et la coque, est en matériau dur.

La cupule étant généralement métallique, une telle liaison convient bien pour l'insertion dans cette cupule d'une coque également en métal ou en céramique.

En revanche, elle n'est pas appropriée pour l'insertion d'un matériau relativement souple tel que le polyéthylène (ou une matière plastique similaire), qui a tendance à fluer et à se déformer lorsque la prothèse est soumise à des efforts, ce qui risque d'entraîner à terme une modification géométrique de la pièce la plus malléable, ce phénomène pouvant être extrêmement préjudiciable au fonctionnement de l'ensemble.

Pour pallier cette difficulté, il a été proposé de pourvoir l'embouchure de la cupule, ou d'une pièce métallique solidaire de la cupule, soit d'une gorge, soit d'un bourrelet périphérique, tandis que la paroi externe de la coque en polyéthylène est pourvue inversement, soit d'une lèvre pouvant se déployer et s'engager dans ladite gorge, soit d'une gorge pouvant recevoir ledit bourrelet.

Des assemblages de ce genre, de type « clipsage » sont notamment décrits dans les documents EP 1 195 149 et EP 1 133 958.

Ce genre de solution est relativement complexe et onéreux.

Le simple engagement de la lèvre ou du bourrelet dans sa gorge réceptrice ne permet pas une immobilisation parfaite de la coque par rapport à la cupule, en translation d'une part, ainsi qu'en rotation. En effet, le déploiement de la lèvre mobile à l'intérieur de la gorge implique un jeu minimum pour le mouvement de la lèvre mobile, ce jeu étant aggravé par la rétractation de la lèvre. En rotation, des micromouvements relatifs restent possibles. Ces différentes mobilités sont susceptibles de générer des microparticules indésirables de polyéthylène, néfastes à la pérennité de la prothèse.

Dans le document WO 95/22944, il est préconisé de monter la coque en polyéthylène à l'intérieur d'une coquille métallique, laquelle peut être fixée par emmanchement par cône morse dans la cupule acétabulaire.

La solidarisation de la coque avec cette coquille intermédiaire requiert la mise en oeuvre d'un procédé thermique (rétreint du polyéthylène par refroidissement lors du montage) complété par une coopération bourrelet/gorge.

Cette technique de fabrication est onéreuse, et le prix de revient du cotyle, comportant une telle coquille intermédiaire, est élevé.

D'un point de vue chirurgical, en cas de nécessité de retrait de la coque ainsi décrite, il apparaît une difficulté majeure de désolidarisation de de la coquille métallique par rapport à la cupule elle-même liée à l'os.

L'invention vise à résoudre ces différents problèmes en proposant un cotyle prothétique de la hanche du genre indiqué plus haut, de conception simple, peu coûteux, et dont la cupule acétabulaire peut être facilement garnie, selon le choix du chirurgien, soit d'une coque en matériau dur (métal ou céramique notamment), soit d'une coque en matériau relativement souple (polyéthylène notamment), leur solidarisation interdisant tout micromouvement relatif entre la cupule et la coque, et n'induisant pas, ou pratiquement pas, de risque de fluage de la matière de la coque après que la prothèse ait été implantée.

L'objet de l'invention est donc un cotyle prothétique articulaire notamment pour articulation de la hanche ou de l'épaule, qui comporte :
- une cupule en matériau dur, ayant une forme générale sensiblement hémisphérique, à symétrie de révolution autour d'un axe -dit central- et présentant une face externe convexe adaptée pour être insérée et fixée dans la cavité cotyloïdienne de l'os, tandis que sa cavité interne présente une zone de fond bordée par une embouchure à paroi légèrement conique, s'évasant vers l'extérieur ;
- une coque en polyéthylène, ou en matériau similaire, apte à être emboîtée suivant ledit axe central à l'intérieur de ladite cupule, de manière à prendre appui par une zone de sa face externe convexe contre ladite zone de fond centrale, cette coque présentant une cavité interne sensiblement hémisphérique destinée à recevoir la tête d'une prothèse articulaire, et étant munie sur sa face externe, d'au moins une saillie déformable.

Conformément à l'invention, ce cotyle est **caractérisé en ce que** :
- d'une part, la portion de la paroi externe de la coque tournée vers l'intérieur de la cupule, est ainsi conformée et dimensionnée que lorsque la coque est emboîtée à l'intérieur de la cupule, il existe un espace annulaire libre ménagé à la périphérie de cette portion et en regard de la paroi conique de ladite embouchure, tandis que ladite saillie est positionnée en vis-à-vis de la base de cette embouchure ;
- d'autre part, le cotyle prothétique comprend également une bague de blocage en matériau dur, apte à être solidarisée avec ladite cupule après avoir été emmanchée axialement, suivant ledit axe central, dans ledit espace annulaire libre, afin de déformer ladite saillie et l'appliquer intimement, en la comprimant, contre la paroi de la cavité interne de la cupule de manière à assurer la solidarisation par friction de la coque avec cette dernière.

La pression de la saillie contre la paroi interne de la cupule, à la base de l'embouchure, assure durablement une excellente immobilisation de l'insert par suite de la pression exercée par la bague elle-même immobilisée dans la cupule.

Il convient de mentionner qu'il a déjà été proposé dans le document US 4 871 368 de solidariser une coque en polyéthylène avec une cupule métallique en faisant usage d'une bague de serrage filetée. Celle-ci est vissée dans un taraudage de l'embouchure de la cupule de manière à s'appliquer contre une nervure annulaire périphérique dont est munie la coque, et à pincer cette nervure entre le chant interne de la bague et un épaulement ménagé dans la cupule.

Dans cette solution, la cupule ne possède pas d'embouchure à paroi tronconique susceptible de recevoir une coque rigide par un assemblage à cône morse.

Dans un mode de réalisation préféré de la présente invention, ladite bague de blocage possède une paroi externe de forme légèrement conique, apte à permettre sa solidarisation avec la cupule par effet de cône morse à l'intérieur de son embouchure.

Cet agencement ne requiert donc pas d'aménagement spécifique de la cupule, tel que la présence d'une gorge par exemple, en vue de l'insertion de l'insert sous forme de coque en matériau souple.

En effet, l'embouchure de cupule à paroi conique prévue pour l'insertion d'une coque rigide, en métal ou en céramique, est également utilisée ici pour l'insertion d'une coque souple, notamment en polyéthylène, grâce à l'interposition de la bague de blocage.

Par ailleurs, selon un certain nombre de caractéristiques additionnelles, non limitatives de l'invention:
- ladite saillie est constituée par une partie en surépaisseur de la paroi externe de la coque qui s'étend, complètement ou partiellement, sur une circonférence de celle-ci ;
- ladite saillie est une lèvre qui s'étend, complètement ou partiellement, sur une circonférence de la paroi externe de la coque, et cette dernière présente une gorge périphérique annulaire qui s'étend à la base de la lèvre de manière à en accroître la déformabilité à la flexion ;
- la bague de blocage et la coque présentent des moyens d'engagement complémentaires, aptes à assurer positivement leur immobilisation mutuelle en rotation par rapport audit axe central ; cette solidarisation en rotation de la coque avec la bague de blocage, et corrélativement avec la cupule qui en est solidaire, réduit encore les risques de micromouvements et de fluage de la coque ;
- d'une part, ladite partie en surépaisseur s'étend de manière continue ou quasi-continue sur une circonférence de la paroi externe de la coque, tandis que, d'autre part, le bord de la bague de blocage (4) tourné vers le fond de la cupule est pourvu d'aiguilles aptes à pénétrer dans le matériau constituant ladite coque lorsque la bague de blocage est emmanchée dans la cupule, assurant ainsi l'immobilisation relative de la bague de blocage et de la coque, en rotation autour de l'axe central ;
- d'une part, la lèvre s'étend de manière discontinue sur une circonférence de la paroi externe de la coque, de sorte qu'elle est composée de segments de lèvre séparés par des zones lisses, tandis que, d'autre part, le bord de la bague de blocage tourné vers le fond de la cupule a une forme crénelée, pourvue de languettes ainsi conformées qu'elles se positionnent en regard desdites zones lisses en s'encastrant entre deux segments de lèvre voisins lorsque la bague de blocage est emmanchée dans la cupule, assurant ainsi l'immobilisation relative de la bague de blocage et de la coque, en rotation autour de l'axe central ;
- les différentes languettes et les différentes zones lisses ont des longueurs circonférentielles égales et sont réparties angulairement de manière régulière autour de l'axe central ;
- après mise en place de la coque et de la bague de blocage dans la cupule, les chants extérieurs de la coque, de la bague de blocage, et de la cupule, s'inscrivent sensiblement dans un même plan qui est perpendiculaire à l'axe central ;
- la cavité interne de la cupule présente un fond comprenant une zone centrale sensiblement plane, perpendiculaire audit axe central, et une zone périphérique à paroi sensiblement en forme de portion de sphère, centrée sur cet axe central, tandis que la paroi de fond de la coque (insert) possède des zones de forme complémentaire, assurant son assise à l'intérieur de la cupule.

Grâce à cette disposition, l'ensemble de la face externe du fond de la coque (insert) est parfaitement soutenu par la paroi interne du fond de la cupule, ce qui permet à cette dernière d'absorber les efforts qui se développent à l'usage dans l'articulation reconstituée, pratiquement sans risque de fluage du polyéthylène, ou autre matériau utilisé comme insert et/ou surface de frottement.

Un tel cotyle trouve notamment application pour l'articulation de la hanche, sa cupule étant alors adaptée pour être insérée et fixée dans la cavité cotyloïdienne de l'os du bassin, tandis que sa coque reçoit la tête d'une prothèse fémorale.

Il peut également s'appliquer à l'articulation de l'épaule, sa cupule étant adaptée dans ce cas pour être insérée et fixée dans la glène de l'omoplate, tandis que sa coque reçoit la tête d'une prothèse humérale.

D'autres caractéristiques et avantages de la présente invention apparaîtront à la lumière de la description qui va maintenant en être faite, en référence aux dessins annexés.

Ces dessins représentent, à titre d'exemple non limitatif, deux modes d'exécution du cotyle prothétique faisant l'objet de l'invention.
La figure 1 est une vue éclatée et en perspective montrant, avant assemblage, les trois éléments constitutifs du cotyle selon un premier mode de réalisation.
La figure 2 est une vue éclatée similaire, mais en coupe axiale.
La figure 3 est une vue de dessous de la bague de blocage.
La figure 4 est une vue de dessus de la coque.
La figure 5 est une vue analogue à celle de la figure 1, après assemblage des trois éléments constitutifs du cotyle.
La figure 6 est une vue éclatée et en perspective montrant, avant assemblage, les trois éléments constitutifs du cotyle selon un second mode de réalisation.
La figure 7 est une vue analogue à celle de la figure 6, après assemblage de trois éléments constitutifs du cotyle.

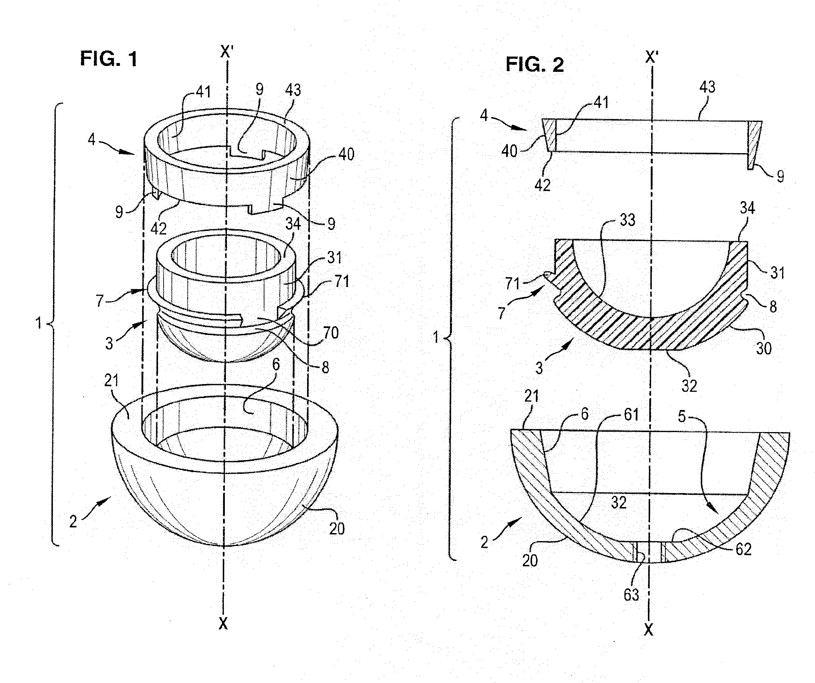

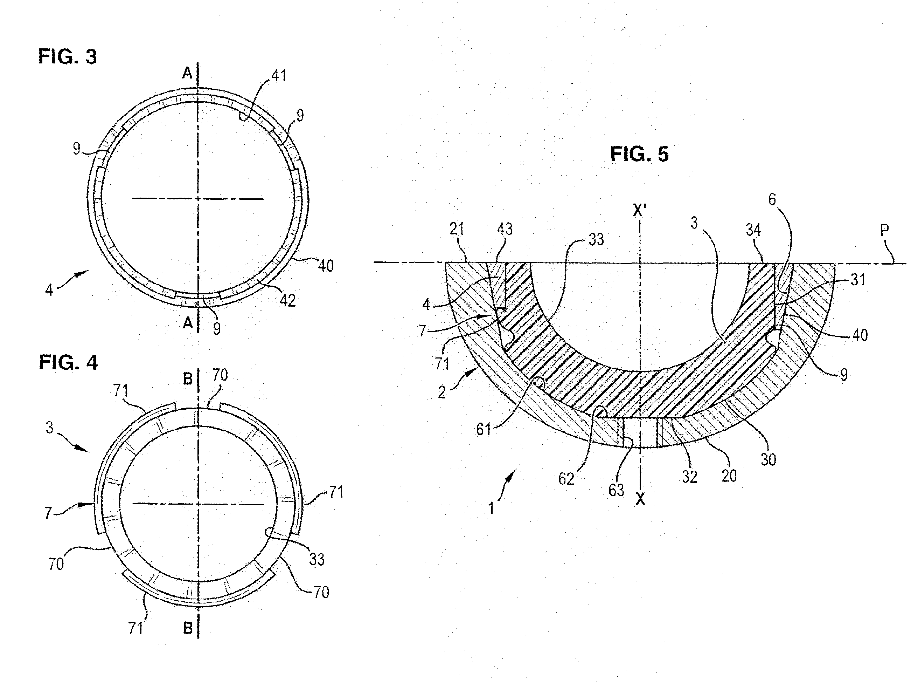

Les plans référencés respectivement **A-A** et **B-B** sur les figures 3 et 4 correspondent au plan de section des figures 2 et 5.

Un premier mode de réalisation de l'invention va maintenant être décrit en liaison avec les figures 1 à 5.

Comme cela est notamment visible sur les figures 1 et 2, le cotyle prothétique illustré, qui porte la référence 1, est composé de trois pièces, à savoir une cupule 2, une coque 3 et une bague 4.

La cupule 1 est rigide, de préférence en métal ou en un autre matériau dur et biocompatible.

Il s'agit d'une pièce de révolution, d'axe de symétrie **X-X'**, que l'on appellera « axe central ».

Sa face externe 20 est convexe, par exemple à surface sphérique, adaptée pour se loger dans la cavité cotyloïdienne de l'os du bassin, et y être fixée.

De façon connue, cette fixation peut être réalisée au moyen de plusieurs vis qui traversent des orifices adéquats percés radialement dans la paroi de la cupule, ces orifices pouvant être chanfreinés pour noyer la tête fraisée de la vis qui les traverse. En outre, la face externe 20 peut être garnie de nervures, dents, picots ou autres saillies, aptes favoriser l'immobilisation de la cupule dans l'os.

De telles dispositions, bien connues en soi, ne font pas à proprement parler partie de l'invention et, en conséquence, n'ont pas représentées ici afin de ne pas alourdir inutilement la description ni les dessins.

Comme on le voit sur la figure 2, cette cupule 2 présente une cavité interne 5, composée d'une paroi de fond 61-62 et d'une paroi d'embouchure 6.

La paroi 6 est tronconique, s'évasant vers l'extérieur de la cupule, avec une faible conicité, permettant un assemblage par effet de cône morse avec une pièce de forme complémentaire rigide, notamment en métal ou en céramique (non représentée). L'angle au sommet de cette paroi tronconique est par exemple de l'ordre de l'ordre de 6° à 8 ° (soit une valeur de 3 à 4° pour le demi-angle au sommet).

La paroi de fond présente une zone centrale plane 62, perpendiculaire à l'axe central **X-X'**, qui est bordée par une zone concave 61, à surface sphérique ou approximativement sphérique.

La paroi de fond est traversée par un trou axial taraudé 63, dont la fonction est de permettre la manipulation et le positionnement de la cupule 2 dans l'os du bassin à l'aide d'une tige d'ancillaire à extrémité filetée, ceci selon une disposition connue en soi.

La référence 21 désigne le chant extérieur de la cupule 2.

La coque 3 est en matière plastique, de préférence en polyéthylène à haute densité, du genre couramment utilisé dans la présente application.

La coque 3 peut également être réalisée dans un matériau similaire au polyéthylène. Par "similaire", on entend tout matériau biocompatible, ayant des propriétés mécaniques lui permettant de fonctionner et de se déformer comme décrit ci-après.

Elle présente une cavité interne 33 sensiblement hémisphérique, d'axe de symétrie **X-X'**, adaptée pour loger la tête quasi-sphérique de la prothèse fémorale (non représentée).

Sa face externe comprend une portion de fond convexe 30-32 complémentaire de celle de la cavité 5, de sorte qu'après montage ses portions galbée 30 et plane 32 viennent prendre appui et reposer respectivement contre les portions 61 et 62 de la cupule 5 (voir figure 5).

Cette portion de fond se prolonge vers l'extérieur par une portion cylindrique 31, dont la hauteur (dimension suivant l'axe **X-X'**) est sensiblement égale à celle de la portion tronconique 6 d'embouchure de la cupule 2.

Entre la zone de fond 30 et la portion 31, la face externe de la coque 2 présente (côté fond) une gorge annulaire 8, par exemple à profil semi-circulaire, bordée (côté extérieur) par une lèvre annulaire 71, par exemple à profil triangulaire.

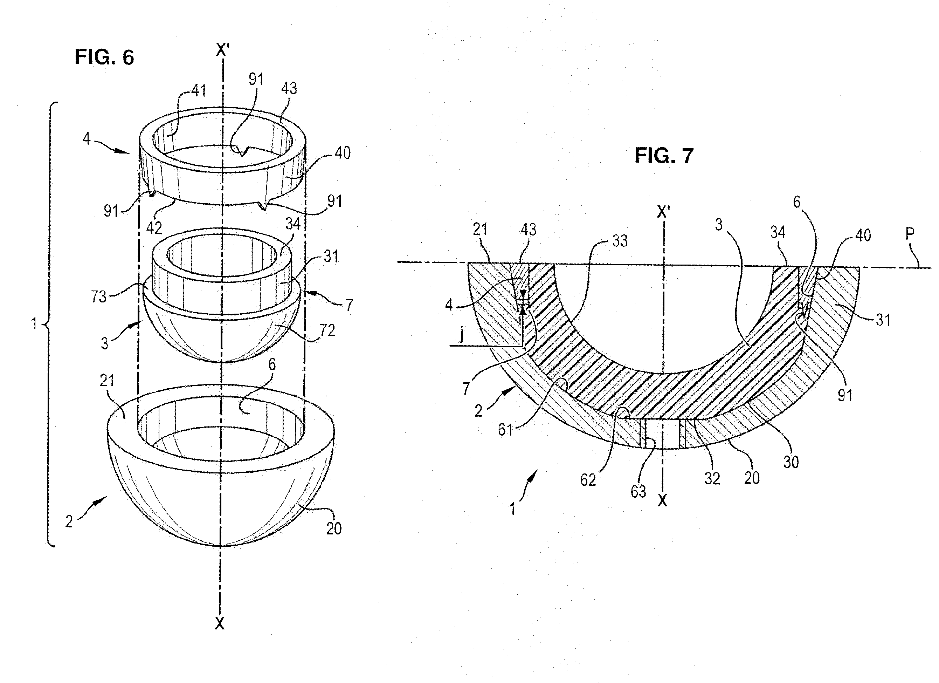

Cette lèvre 71 constitue un premier mode de réalisation d'une saillie 7, cette dernière se retrouvant également sur le second mode de réalisation qui sera décrit ultérieurement en liaison avec les figures 6 à 7.

En raison de la souplesse et de l'élasticité de la matière constitutive de la coque 3, cette lèvre peut fléchir vers l'extérieur et du côté du fond (déformation élastique), dès lors qu'elle est soumise à un effort axial dirigé vers le fond. La capacité de déformation à la flexion de la lèvre 71 se trouve accrue grâce à la présence de la gorge 8 creusée juste à sa base.

Comme on le voit sur les figures 1 et 4 notamment, la lèvre 71 est discontinue ; elle est composée de trois segments de même longueur circonférentielle, séparés par des zones lisses 70 de moindre longueur circonférentielle. Ces segments, ainsi que les zones lisses, sont régulièrement réparties à 120° autour de l'axe central.

La référence 34 désigne le chant extérieur de la coque 3.

La bague de bocage 4 est en matériau dur et rigide, avantageusement en métal.

Elle a la configuration générale d'un tronçon de tube d'axe **X-X'**, dont la paroi interne 41 est cylindrique et la paroi externe 40 légèrement conique, convergeant du côté du fond de cupule 5.

Le diamètre intérieur de la bague 4 est égal, au jeu d'emmanchement près, au diamètre extérieur de la portion cylindrique 31 de la coque 3.

La conicité de sa paroi externe 40 est la même - ou sensiblement la même - que celle de la paroi d'embouchure 6 de la cupule 2.

La référence 43 désigne le chant extérieur de la bague 4.

Son bord intérieur 42 (chant tourné du côté du fond de cupule) est discontinu, avec un contour crénelé par la présence de trois languettes 9 qui en prolongent la paroi axialement, sur une faible hauteur.

Ces languettes 9 ont la même disposition angulaire, à 120°, et la même longueur circonférentielle, que les zones lisses 70 susmentionnées.

Lorsque le chirurgien implante le cotyle 1, il commence par insérer et positionner correctement la cupule 2 dans l'os du bassin, puis l'y fixe au moyen d'un ciment et/ou de vis.

Dès lors qu'il souhaite utiliser une coque en métal ou en céramique, il l'encastre de manière classique dans la cupule 2 afin d'obtenir une solidarisation mutuelle de ces deux pièces par effet de cône morse.

Dès lors qu'il souhaite utiliser la coque en polyéthylène 3, il emboîte cette dernière dans la cavité 5 de la cupule 2 de telle façon que ses portions de paroi de fond 32-30 viennent en appui respectivement contre les zones de fond de cupule 62, respectivement 61. La coque et la cupule sont alors parfaitement centrées l'une par rapport à l'autre, suivant l'axe central **X-X'**.

Du fait que la paroi externe 31 de l'embouchure de la coque 3 est cylindrique et possède un diamètre inférieur à celui de la paroi d'embouchure 6 de la cupule, il existe un jeu annulaire entre ces deux parois.

La bague 4 possède une section trapézoïdale dont la forme et les dimensions sont identiques à celles de la section de cet espace libre.

Le chirurgien commence par positionner la bague 4 à l'entrée dudit espace libre, suivant l'axe central **X-X'**, l'oriente correctement par pivotement autour de cet axe de sorte que les languettes 9 se placent chacune en vis-à-vis d'une zone lisse 70, puis l'emmanche axialement à l'intérieur de la cupule, autour de la paroi 31 de la coque. Dans le but d'obtenir le même résultat, le chirurgien peut, préalablement à la mise en place de la cupule, positionner la bague à section trapézoïdale sur l'insert en polyéthylène, et impacter l'ensemble dans la cupule préalablement fixée dans la cavité cotyloïdienne du bassin.

La hauteur (longueur axiale) de cette bague 4 est choisie de telle sorte qu'en fin de course d'emmanchement, son chant 42 vient presser contre les segments de lèvre 71, en les faisant fléchir vers l'extérieur et vers le fond de la cupule, contre la base de sa portion de paroi conique 6. On obtient ainsi une immobilisation très efficace, par effet de friction élevée au niveau des trois segments de lèvre, de la coque à l'intérieur de la cupule. La compression de la lèvre est permanente et demeure active tout au long de l'usage ultérieur de la prothèse.

Dans le même temps, on obtient une immobilisation, également très efficace, de la bague par rapport à la cupule, grâce son emmanchement de type cône morse dans la paroi d'embouchure 6.

Enfin, chaque languette 9 se trouve encastrées entre deux segments de lèvre 71 ce qui à ce niveau assure un verrouillage anti-rotation de la coque 3 par rapport à la bague 4 et, corrélativement, par rapport à la cupule 2.

Les risques de micro-déplacements entre la coque et la cupule, et de fluage de la paroi de la coque, en cours d'usage ultérieur de la prothèse s'en trouvent considérablement réduits, voire éliminés.

Lorsque l'assemblage est correctement réalisé, les chants 21 de la cupule, 43 de la bague de blocage, et 34 de la coque, se trouvent positionnés dans un même plan transversal (perpendiculaire à **X-X'**), référencé **P** sur la figure 5.

On notera l'extrême simplicité de cet assemblage, qui peut être réalisé à l'aide d'un instrument ancillaire approprié facile à manipuler et à utiliser (outil impacteur par exemple).

Pour faciliter le bon positionnement angulaire de la bague 4 par rapport à la coque 3 avant leur assemblage, des repères peuvent être prévus sur leurs chants respectifs 43 et 34 ; ces repères sont destinés à venir en coïncidence lorsque leur orientation mutuelle est correctement réalisée, avec les languettes 9 en vis-à-vis des zones lisses 70. En outre, ces languettes pourraient avoir de bords biseautés faisant office de chanfreins d'entrée et de centrage, favorisant leur insertion entre deux segments de lèvre voisins.

Différentes variantes d'un tel cotyle peuvent être envisagées, sans qu'elles ne sortent du cadre de la présente invention.

Ainsi, la lèvre 71 pourrait être continue, de forme annulaire courant sur toute la périphérie de la coque 3.

Dans cette hypothèse, le chant 42 serait également continu (et non crénelé), apte à prendre appui et à comprimer cette lèvre sur l'ensemble de sa circonférence.

L'immobilisation relative des deux éléments en rotation pourrait dans ce cas être obtenue par l'engagement de nervures longitudinales (parallèles à **X-X'**) prévues dans la paroi de coque 31 dans des rainures longitudinales réceptrices, de section complémentaire, creusées dans la paroi 41 de la bague 4.

Inversement, les nervures pourraient naturellement être portées par la paroi 41 et les rainures formées dans la paroi 31.

La solidarisation de la bague 4 avec la cupule 2 n'est pas obligatoirement réalisée par effet de cône morse entre les parois 40 et 6; elle pourrait être réalisée par exemple à l'aide de vis.

Ainsi, selon un mode de réalisation possible de l'invention (non représenté), la paroi externe 40 de la bague est cylindrique, de diamètre sensiblement légèrement inférieur à celui de la base de la paroi tronconique 6 de la cupule.

Le bord extérieur de la bague est pourvu d'une collerette annulaire venant prendre appui, en fin de course d'emmanchement, contre le chant 21 de la cupule, ou contre un lamage annulaire formé dans celui-ci, à l'embouchure de la cupule. Ladite collerette est percée de plusieurs ouvertures, par exemple de quatre trous à entrée chanfreinée, d'axe longitudinal (parallèle à **X-X'**) disposés à 90° par rapport à l'axe central; ces ouvertures sont destinées à être traversées chacune par une vis de fixation à tête fraisée, que l'on visse dans un trou taraudé d'axe longitudinal formé dans l'épaisseur de paroi de la cupule, et pour servir de logement à la tête de cette vis.

Un second mode de réalisation de l'invention va maintenant être décrit. Il diffère du premier par la forme de la saillie 7 prévue sur la coque 3 et par la forme de la bague 4.

Les autres éléments sont identiques et ne seront pas décrits en détails de nouveau. Les éléments communs aux deux modes de réalisation portent les mêmes références numériques.

Comme on peut le voir sur la figure 6, la forme de la coque 3 est modifiée en ce que la gorge annulaire 8 et la lèvre 71 ont été supprimées. La saillie 7 est ici constituée par une partie 72 en surépaisseur de la paroi externe de la coque 3. Cette partie en surépaisseur 72 présente un diamètre légèrement supérieur à celui de la portion cylindrique 31, de sorte qu'elle définit avec celle-ci un épaulement 73 annulaire.

La partie en surépaisseur 72 est ménagée sensiblement au centre de la coque 3, c'est-à-dire entre la partie galbée 30 du fond et la portion cylindrique 31.

La partie 72 s'étend sur une partie, ou de préférence sur la totalité de la circonférence de la paroi externe de la coque 3.

En outre, dans ce mode de réalisation, les languettes 9 de la bague 4 sont remplacées par des aiguilles 91. On entend par "aiguille" tout élément tel qu'un pion ou un picot suffisamment pointu et acéré pour pénétrer dans le matériau constituant la coque 3 lorsque la bague 4 est positionnée en regard de ladite coque 3.

Les aiguilles sont au nombre d'au moins une, voire plusieurs réparties de préférence à intervalles réguliers sur la circonférence de la bague 4. Elles sont par exemple au nombre de trois, répartis à 120° ou au nombre de quatre, réparties à 90°.

Lorsque la bague 4 est emmanchée dans l'espace libre existant entre la paroi de l'embouchure 6 et la paroi externe 31 de la coque 3, la ou les aiguilles 91 vient (viennent) presser contre l'épaulement 73 de la portion en surépaisseur 72 en déformant cette partie 72, notamment vers le bas, de façon à la comprimer et à l'appliquer contre la paroi 6 de la cavité interne de la cupule 2.

On obtient ainsi une immobilisation par friction très efficace. Les aiguilles 91 participent également à cette déformation de la partie en surépaisseur 72 lorsqu'elles pénètrent dans le matériau, au niveau de l'épaulement 73. On observe dans ce cas une légère déformation élastique et surtout une déformation plastique de la partie en surépaisseur 72.

Comme on peut le voir sur la figure 7, une fois la bague 4 emmanchée et les aiguilles 91 fichées dans le matériau constituant la coque 3, il existe de préférence un léger jeu j entre le chant 42 et l'épaulement 73.

En outre, la pénétration des aiguilles 91 permet d'assurer l'immobilisation relative de la bague de blocage 4 et de la coque 3, en rotation autour de l'axe central (X-X').

De préférence, la partie en surépaisseur (72) s'étend de manière continue ou quasi-continue sur la circonférence de la paroi externe de la coque 3, de sorte que quelle que soit l'orientation angulaire de la bague 4 autour de l'axe central X-X', on soit assuré que les aiguilles 91 pénètreront dans cette partie en surépaisseur 72.

Cette variante de réalisation permet de ne pas avoir besoin de repères visuels ou de détrompeurs et ne nécessite pas un positionnement précis de la bague 4 comme dans le premier mode de réalisation.

L'invention peut s'appliquer à d'autres types de prothèse, notamment pour l'articulation de l'épaule.

## Revendications

1. Cotyle prothétique articulaire (1), notamment pour l'articulation de la hanche ou de l'épaule, qui comporte :
- une cupule (2) en matériau dur, ayant une forme générale sensiblement hémisphérique, à symétrie de révolution autour d'un axe (**X-X'**) -dit central- et présentant une face externe convexe (20) adaptée pour être insérée et fixée dans la cavité cotyloïdienne de l'os, tandis que sa cavité interne (5) présente une zone de fond (61- 62) bordée par une embouchure (6) à paroi légèrement conique, s'évasant vers l'extérieur ;
- une coque (3) en polyéthylène, ou en matériau similaire, apte à être emboîtée suivant ledit axe central (**X-X'**) à l'intérieur de ladite cupule (2), de manière à prendre appui par une zone de sa face externe convexe (30-32) contre ladite zone de fond centrale (61- 62), cette coque (3) présentant une cavité interne (33) sensiblement hémisphérique destinée à recevoir la tête d'une prothèse articulaire, et étant munie sur sa face externe, d'au moins une saillie (7) déformable ;
**caractérisé par le fait que** :
- d'une part, la portion (31) de la paroi externe de la coque (3) tournée vers l'intérieur de la cupule (2) est ainsi conformée et dimensionnée que lorsque la coque (3) est emboîtée à l'întérieur de la cupule (2), il existe un espace annulaire libre ménagé à la périphérie de cette portion (31) et en regard de la paroi conique de ladite embouchure (6), tandis que ladite saillie (7) est positionnée en vis-à-vis de la base de cette embouchure (6) ;
- d'autre part, le cotyle prothétique (1) comprend également une bague de blocage (4) en matériau dur apte à être solidarisée avec ladite cupule (2) après avoir été emmanchée axialement, suivant ledit axe central (**X-X'**), dans ledit espace annulaire libre, afin de déformer ladite saillie (7) et l'appliquer intimement, en la comprimant, contre la paroi de la cavité interne (5) de la cupule (2) de manière à assurer la solidarisation par friction de la coque (3) avec cette dernière.

2. Cotyle prothétique selon la revendication 1, **caractérisé par le fait que** ladite bague de blocage (4) possède une paroi externe (40) de forme légèrement conique, apte à permettre sa solidarisation avec la cupule (2) par effet de cône morse à l'intérieur de son embouchure (6).

3. Cotyle prothétique selon la revendication 1 ou la revendication 2, **caractérisé par le fait que** ladite saillie (7) est constituée par une partie en surépaisseur (72) de la paroi externe de la coque (3) qui s'étend, complètement ou partiellement, sur une circonférence de celle-ci.

4. Cotyle prothétique selon la revendication 1 ou la revendication 2, **caractérisé par le fait que** ladite saillie (7) est une lèvre (71) qui s'étend, complètement ou partiellement, sur une circonférence de la paroi externe de la coque (3), et que cette dernière présente une gorge périphérique annulaire (8) qui s'étend à la base de ladite lèvre (7) de manière à en accroître la déformabilité à la flexion.

5. Cotyle prothétique selon l'une quelconque des revendications 1 à 4, **caractérisé par** le fait ladite bague de blocage (4) et ladite coque (3) présentent des moyens d'engagement complémentaires, aptes à assurer leur immobilisation mutuelle en rotation par rapport audit axe central (X-X').

6. Cotyle prothétique selon les revendications 3 et 5, **caractérisé par le fait que**, d'une part, ladite partie en surépaisseur (72) s'étend de manière continue ou quasi-continue sur une circonférence de la paroi externe de la coque (3), tandis que, d'autre part, le bord de la bague de blocage (4) tourné vers le fond de la cupule (2) est pourvu d'au moins une aiguille (91) apte à pénétrer dans le matériau constituant ladite coque (3) lorsque la bague de blocage (4) est emmanchée dans la cupule (2), assurant ainsi l'immobilisation relative de la bague de blocage (4) et de la coque (3), en rotation autour de l'axe central (X-X').

7. Cotyle prothétique selon les revendications 4 et 5, **caractérisé par le fait que**, d'une part, ladite lèvre s'étend de manière discontinue sur une circonférence de la paroi externe de la coque (3), de sorte qu'elle est composée de segments de lèvre séparés par des zones lisses (70), tandis que, d'autre part, le bord de la bague de blocage (4) tourné vers le fond de la cupule (2) a une forme crénelée, pourvue de languettes (9) ainsi conformées qu'elles se positionnent en regard desdites zones lisses (70) en s'encastrant entre deux segments de lèvre voisins lorsque la bague de blocage (4) est emmanchée dans la cupule (2), assurant ainsi l'immobilisation relative de la bague de blocage (4) et de la coque (3), en rotation autour de l'axe central (X-X').

8. Cotyle prothétique selon la revendication 7, **caractérisé par le fait que** les différentes languettes (9) et les différentes zones lisses (70) ont des longueurs circonférentielles égales et sont réparties angulairement autour dudit axe central (X-X').

9. Cotyle prothétique selon l'une quelconque des revendications 1 à 8, **caractérisé par le fait qu'**après mise en place de la coque (3) et de la bague de blocage (4) dans la cupule (2), les chants extérieurs (34) de la coque (3), (43) de la bague de blocage (4), et (21) de la cupule (2), s'inscrivent sensiblement dans un même plan (P) qui est perpendiculaire audit axe central (X-X').

10. Cotyle prothétique selon l'une quelconque des revendications 1 à 9, **caractérisé par le fait que** la cavité interne (5) de la cupule présente un fond comprenant une zone centrale (62) sensiblement plane, perpendiculaire audit axe central (X-X'), et une zone périphérique (61) à paroi sensiblement en forme de portion de sphère, centrée sur cet axe central (X-X'), tandis que la paroi de fond de la coque (3) possède des zones (32, 30) de forme complémentaire, assurant son assise à l'intérieur de la cupule.
